# EUROPEAN PATENT SPECIFICATION

(11) **EP 1 983 960 B1**
(45) Date of publication and mention of the grant of the patent: **28.10.2015**
(21) Application number: 07703172.2
(22) Date of filing: 31.01.2007
(51) Int. Cl.: A61H 3/00, A61F 5/01, A61H 1/02

(54) **ORTHOPAEDIC APPARATUS FOR WALKING AND REHABILITATING MOTION-INJURED PERSONS**
ORTHOPÄDISCHES GERÄT ZUM GEHEN UND REHABILITATION VON BEWEGUNGSEINGESCHRÄNKTEN PATIENTEN
APPAREIL ORTHOPEDIQUE POUR FAIRE MARCHER ET REHABILITER DES PERSONNES ACCIDENTEES

(30) Priority: 03.02.2006 IT MI20060187
(43) Date of publication of application: 29.10.2008
(73) Proprietor: Cantoni, Luciana, 35100 Padova (IT)
(72) Inventor: FERRATI, Benito, deceased (IT)
(74) Representative: Arena, Giovanni
(86) International application number: PCT/EP2007/000835
(87) International publication number: WO 2007/088044

(56) References cited:
- IT-B- 1 178 548
- IT-B1- 1 298 080
- US-A- 4 651 719
- US-A- 5 961 541
- US-B1- 6 821 233

## Description

The present invention concerns an orthopaedic apparatus for walking and rehabilitating motion-injured persons.

### STATE OF THE ART

For rehabilitating persons injured in the mobility of their lower limbs (paraplegics, etc.), orthopaedic apparatus has been developed to allow perambulating by an exoskeleton moved by micro-cylinders, for the purpose of improving not only their muscular, nervous and osseous condition, but also and above all for stimulating and promoting the recovery of their motor capabilities.

Orthopaedic apparatus of this type, capable of stimulating and exercising the motion of the lower limbs and of allowing perambulation, have been conceived by the same author of this invention, and have been the object of the Italian patents for invention no. 1.153.225, 1.178.548, 1.273.712, 1.281.584 and 1.298.080, of the Italian utility model patent no. 226.083, of the European patents EP 0 782 843 and EP 0 911 015, of the U.S. patent no. 5.961.541 and of the patent application PCT/EP02/12283.

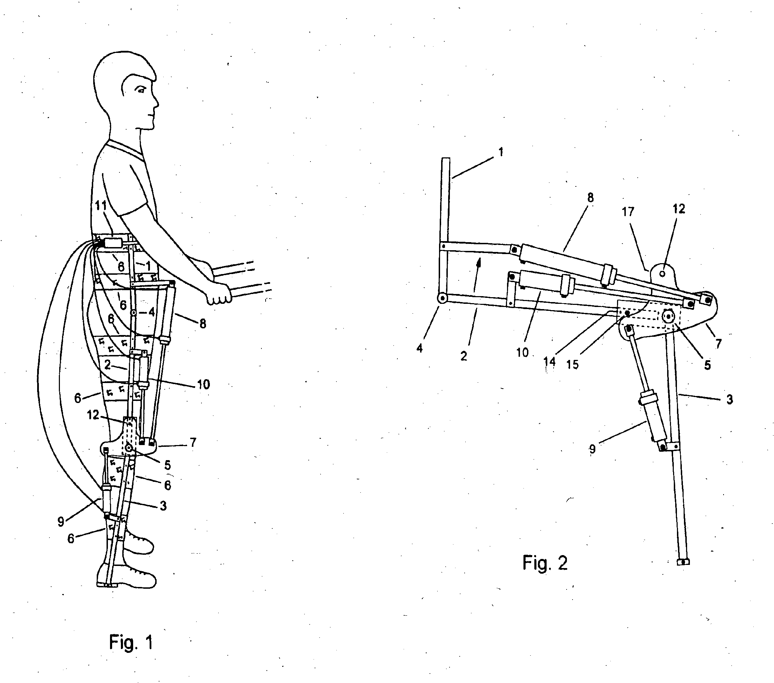

The Italian patent 1.298.080 refers to an orthopaedic apparatus according to the preamble of claim 1. In particular it has faced and resolved the problem of allowing patients to wear an exoskeleton even in a seated condition and to move from an upright to a seated condition and vice-versa, while continuing to wear the exoskeleton controlled by micro-cylinders. Further details on the apparatus as an object of the above patent will be illustrated in the following, with reference to the Figures 1-2.

As even discussed and illustrated in EP 0 782 843 (see relevant Fig.1), the Italian patent no. 1.178.548 discloses an orthopedic apparatus for walking and rehabilitating a motion-injured person with an exoskeleton to support the patient's body, which uses only two actuators to move the lower jointed parts of the exoskeleton in accordance with the human gait. Nevertheless both the choice of the actuators (cylinders operated hydraulically or by compressed air) and the choice of the exoskeleton points which the actuators are hinged to (see in particular the lower actuators capable to impress movements to the patient's legs, whose hinge attachment to the rods set along the patient's thighs is too close to the hinge that connects the same rods with the rods set along the patient's legs) make the apparatus insuitable to hold the entire weight of the patient, who therefore has to lean with his hands to two fixed bars during the rehabilitating exercises.

### SCOPE OF THE INVENTION

The present invention aims at enhancing the apparatus described in Italian patent 1.298.080, with special reference to:
- substantially simplifying the exoskeleton's motion mechanism, by minimizing the number of the relative actuators with a consequent reduction of the exoskeleton's weight and of its encumbrance on the flanks of the patient, and a greater ease of the exoskeleton's usage even as concerns perambulating through corridors or doors of limited width,
- facilitating the patient's own putting on of the apparatus,
- rendering the operation of the apparatus quite noiseless thanks to the particular type of electrically driven actuators employed.

### SUMMARY OF THE INVENTION

The orthopaedic apparatus according to the invention provides for this purpose an exoskeleton to support the patient's body, comprising:
- first, second and third articulated rods (1, 2, 3) set along the patient's trunk, thighs and lower legs respectively and connected by pins (4, 5) opposite the hip and knee joints,
- a pair of plates (6) set externally along one and the other knee joint, respectively,
- first and second actuators (7, 8) anchored by hinges (9, 10, 11, 12) to said plates (6) and said articulated rods (1, 3), respectively, capable of imparting motions on said rods corresponding to those of a human gait,
- an electronic control unit connected to said actuators to control their operation, so as to impart said motions to said articulated rods,
- a remote control for actuating said control unit by the patient, providing the possibility of sending, to said control unit, in addition to perambulating commands, also commands allowing the patient to assume an upright or a seated position,
- a bodice (13) fitted to the patient's trunk, of a partially rigid structure, capable of rigidly connecting the first articulated rods (1),
- means capable of clasping and rigidly fastening the first articulated rods flanking the patient's trunk to the sides of the bodice, at the time the bodice is put on by the patient,
- the hinges (10, 11) present on each of said plates (6) are flanked at the two sides of said second articulated rods (2),
characterized in that:
- each of said plates (6) is firmly attached to the one and the other of said second articulated rods (2) set along the patient's thighs, at a point of the rod destined to align itself with the patient's knee,
- the hinges (12) anchoring said second actuators (8) to the third articulated rods (3) are positioned at a point of the rod destined to be flanked at the side of the patient's ankle,
- said actuators are of a mechanical cylinder type, fitted internally with an electric motor, an epicycloidal reducer, and a recirculating ball screw for a linear actuation of the cylinder pushing/pulling rod or tube,
- said electronic control unit is programmed so as to command, upon receipt of a signal to move from a seated to an upright position, a progressive extension of only said first and second actuators' pushing/pulling tubes, until the articulated rods have reached an aligned position, and vice-versa to command, upon receipt of a signal to move from an upright to a seated position, the progressive re-entry of only said first and second actuators' pushing/pulling tubes, until every contiguous pair of the articulated rods has assumed a relative angular position of about 90°.

### BRIEF DESCRIPTION OF THE DRAWINGS

The characteristics of the invention will become better apparent from the following description dealing with examples of embodiment of a non-limitative nature, made with reference to the attached drawings whose various figures show:
- Figs.1 and 2:: an exoskeleton as shown in the Figures 2 and 5 of the Italian patent no. 1.298.080;
- Fig. 3:: a side view of the exoskeleton's metallic armature according to the invention (right-hand side);
- Fig. 4:: a perspective view of the bodice and right-hand side of the exoskeleton example according to the invention, omitting the actuating cylinders and the plate along the knee joint for viewing simplicity;
- Figs.5A and 5B:: views showing the device fastening the exoskeleton's upper rods to the bodice;
- Fig. 6:: a schematic view of a first example of the exoskeleton's usage according to the invention, showing a patient in an upright position wearing the exoskeleton and grasping the handles of a stroller;
- Fig.7:: a side view of the exoskeleton's armature and of the relative actuators corresponding to a patient's seated position;
- Figs.8A and 8B:: views of further examples of the exoskeleton's usage according to the invention.

### DESCRIPTION OF PREFERRED EMBODIMENTS OF THE INVENTION

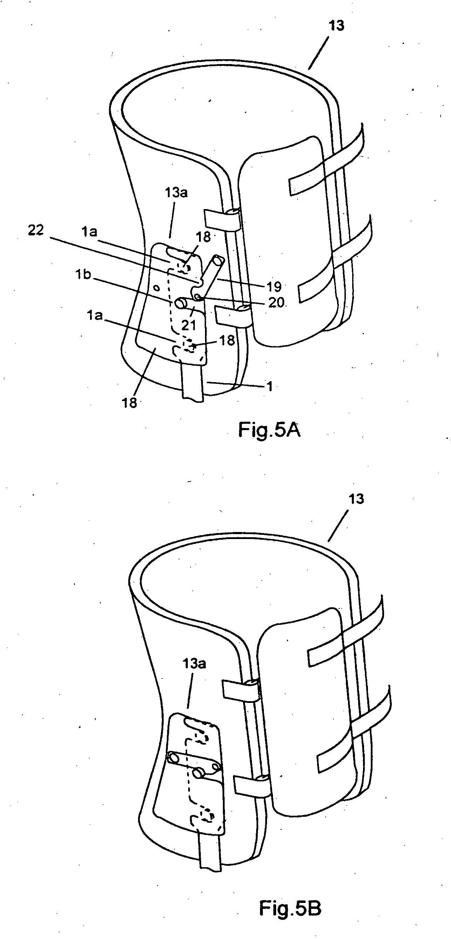

The Figures 1 and 2 (corresponding to the Figure 2 and 5, respectively, of the Italian patent no. 1.298.080) illustrate examples of embodiment of the known exoskeleton, whereof this invention constitutes an improvement.

Figure 1 in particular shows an exoskeleton worn by a stationary patient in an upright position and with his hands grasping the handles of a 4-leg stroller (not shown in the figure), whose front legs are fitted with wheels.

The figure shows a harness or exoskeleton comprising:
- metallic rods 1, 2, 3 flanking the trunk, thighs and legs, respectively, and connected to each other by pins 4 and 5 placed opposite the hip and knee joints and fastened to the patient's body by straps 6 (other rods not seen in the figure are present at the insides of thigh and leg).
- a plate 7 hinged on the pin 5;
- a first, second and third actuator 8, 9, 10 (each composed for example of a compressed air-driven micro-cylinder) hinged at their one end to said plate, and at the other end to shelf-like extensions attached to the rods 1, 3 and 2, respectively,
- a switch box 11 housing valves for power supply and operating of the the micro-cylinders,
- an electronic control unit (not shown in the figure) to control the electrical valves,
- a manually operated remote control (not shown in the figure) that transmits commands for operating the exoskeleton to said electronic unit, with push buttons provided on the handles of the stroller.

In the position of Fig. 1 the exoskeleton is ready to start the perambulating motions. In this position and while perambulating the plate 7 (turning around the pin of hinge 5) is firmly anchored to the rod 2 by a small clasping pin engaging in a hole 12 of the same plate.

To start the perambulation, the patients presses a pushbutton on the remote control (provided on the handle) which has been especially provided to induce, at each push, an alternating step on one of the two legs. As a result of this push, the micro-cylinders 8 and 9 (and alternatively the corresponding micro-cylinders not visible in the figure, placed on the patient's left flank) move the rods 2 and 3 so as to induce the leg to move by one step.

If on the other hand the patient wishes to move from the upright position in Fig. 1 to a seated position (on a chair set up in back of him), he actuates another appropriate button on the remote control. This operation deactivates the micro-cylinders 8 and 9 and activates a small micro-cylinders 15 beneath the hooking pin that causes the same to be extracted from the hole 12. This determines a progressive re-entering motion of the rod of micro-cylinders 8, and a progressive extending motion of the rod of the micro-cylinder 10, and a rotation of the plate 7 with respect to the rod 2, until the exoskeleton has attained the seated position of Fig. 2.

If the patient wishes to resume the upright position starting from the seated position, he again grasps the stroller handles and operates a remote control button, which has been especially provided at this point to motion from a seated to an upright position. Upon operating the latter button, the operations previously described for moving from an upright to a seated position repeat themselves in a reverse order.

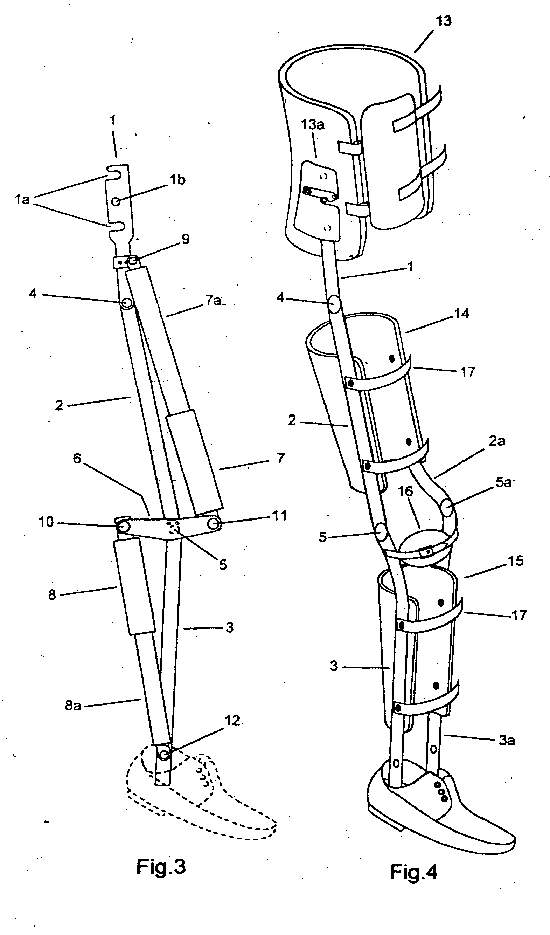

The Figures 3 and 4 relate to an example of an embodiment of the orthopaedic apparatus according to the invention.

In particular, Fig. 3 schematically shows the exoskeleton's metallic armature with the relative actuators viewed from its right hand side, while Fig. 4 shows a perspective view of the exoskeleton's right hand side with the relative bodice and legging (for viewing simplicity, the actuators and plate 6 have been omitted from Fig. 4). A similar disposition of the armature, bodice and leggings also applies to the left-hand side.

The various parts designated by numbers in Fig. 3 stand for the following:
- 1,2,3:: metallic rods destined to align themselves (on the right hand side) with the patient's trunk, thigh and leg;
- 4, 5:: pins connected to said rods and destined to align themselves with the hip joint and knee joint, respectively;
- 6:: a plate destined to align itself with the knee and attached to the rod 2;
- 7, 8:: actuators having extremities anchored to the rods 1 and 3 and to the plate 6 through hinges 9, 12, 10 and 11, respectively; said actuators are of a mechanical cylinder type incorporating an electrical motor (powered by 24 VDC), an epicycloidal reducer and a recirculating ball screw for the linear actuation of the cylinder's pushing (and pulling) tubes or rods (tubes 7 and 8a of Fig. 3); the pushing cylinder and tube are made of aluminium.

The operation of the actuators presupposes the presence of:
- an electrical power source for the same,
- an electrical control unit capable of commanding their operations so as to impart relative motions to the articulated rods in accordance with the human step,
- a remote control for actuating the mentioned electronic control unit by the patient.

According to a preferred solution, the hinge of lower rod (destined to come along the patient's leg) anchored to the relative actuator is positioned at a point of the rod destined to be flanked at the side of the patient's ankle so as to minimize the forces applied by the actuator during its operation.

According to another preferred solution, the upper part of the rod 1 destined to be flanked at the side of the patient's trunk has the shape of a plate with two loops 1 a and a pin 1 b, where said plate is destined to couple with a removable anchoring device present on the exoskeleton bodice, as explained in the following, with reference to the Figures 4, 5A and 5B.

In Fig. 4 the various reference numbers indicate:
- 13:: a bodice with a form fitting the trunk of the patient, of an at least partially rigid structure, so as to ensure a rigid connection between the right and left sides of the exoskeleton's metallic armature when put on; the bodice is preferably made in a single unit of a rigid material internally coated with a layer of soft and elastic material (such as for instance rubber latex);
- 13a:: a clasping device, capable of clasping and fastening the exoskeleton rods, in a removable manner, along the patient's trunk at the time the exoskeleton is put on by the patient;
- 2a, 3a:: rods destined to be flanked at the inner side of the patient's thigh and leg, respectively, and connected to each other by a hinge 5a;
- 14, 15:: leggings made preferably of the same material composing the bodice, affixed to the two sides of the rods 2, 2a and 3, 3a, respectively and destined to be tied to the patient's thighs and legs by straps 17;
- 16:: a belt with a buckle having its ends attached to the upper ends of the rods 3 and 3a and destined to be tightened against the patient's knee, so as to withstand the forces imposed by the patient's limb on the exoskeleton at the time of moving from a seated to an upright position, and vice-versa.

The Figures 5A and 5B illustrate the anchoring system of the bodice 13 to the rod 1 in greater detail.

The Figure 5A shows a metallic plate 18 fastened to the bodice 13 and offering on its front side a hollow space (not visible in the figure) of adequate width to allow the entrance of the upper end of the rod 1, and holding two pins 18 capable of engaging with the loops 1a of the mentioned rod. The outer wall of the hollow space also offers a loop 21 of a length adequate to allow the entrance of the pin 1 b of the same rod.

Once inserted into the mentioned hollow space, the upper part of the rod 1 can be locked in this position by a crank 19 turning around a pin 20, whose counter-clockwise rotation engages a recess of its own 22 with the mentioned pin 1 b. The figure 5B shows the crank 19 in such a locking position.

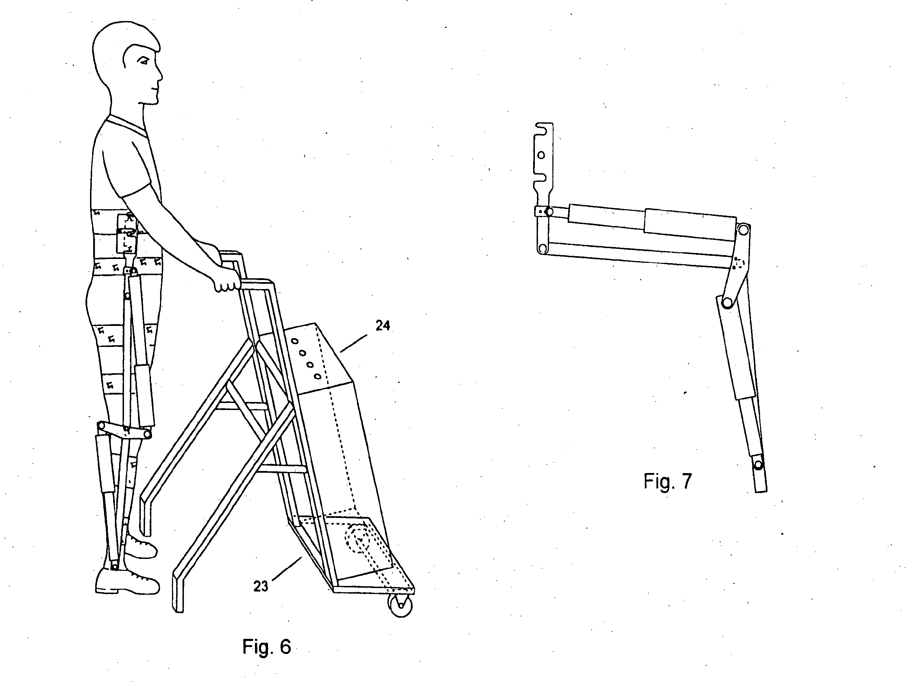

The Figure 6 shows one exoskeleton usage mode. This mode concerns an use of the exoskeleton together with a four-legged stroller 23, whose front legs rest on wheels and which carries a box 24 containing the actuator feeding batteries and the electronic control unit for the same.

Push-buttons for the mentioned remote control (not visible in the figure) are provided on the stroller handles, so as to allow the patient to control the perambulation as already explained in relation to the exoskeleton's operation in Figure 1 (For viewing simplicity, Figure 6 omits the electrical connections between the actuator feeding cable outlets 24 and the actuators themselves).

In addition to the control keys for perambulating, the remote control also holds keys suitable for commanding the motion from a patient's seated position to an upright position, and vice-versa. The mentioned electronic control unit is in fact programmed so that upon receipt of a command for moving from a seated to an upright position it commands the progressive extension of the actuators' pushing/pulling tubes, until the articulated rods flanked at the side of the patient's trunk, thighs and legs are brought to an aligned position. Vice-versa, upon receipt of a command for moving from an upright to a seated position, it commands the progressive re-entry of the actuators' pushing/pulling tubes until each adjacent pair of articulated rods flanking the patient's trunk, thighs and legs is brought to an angular position of about 90°.

Figure 7 shows, e.g., the position of the rods and actuators corresponding to a patient's seated position.

The advantages of the present inventive solution compared to the solution in Figures 1 and 2 are evident. In fact the following advantages can be pointed out in present solution:
- the micro-cylinders 10 of the Figures 1 and 2 have been eliminated;
- the turning plate 7 shown in the same figures has been replaced by the new plate 6, which is firmly attached to the rod 2 (Figures 3 and 4);
- the micro-cylinders 15 of Figs.1 and 2 and the relevant clamping pins have been consequently eliminated;
- the elimination of the micro-cylinders 10 of the Figures 1 and 2 also allows reducing the exoskeleton's encumbrance in a lateral direction, so as to permit perambulating in runs of limited width (80 cm).

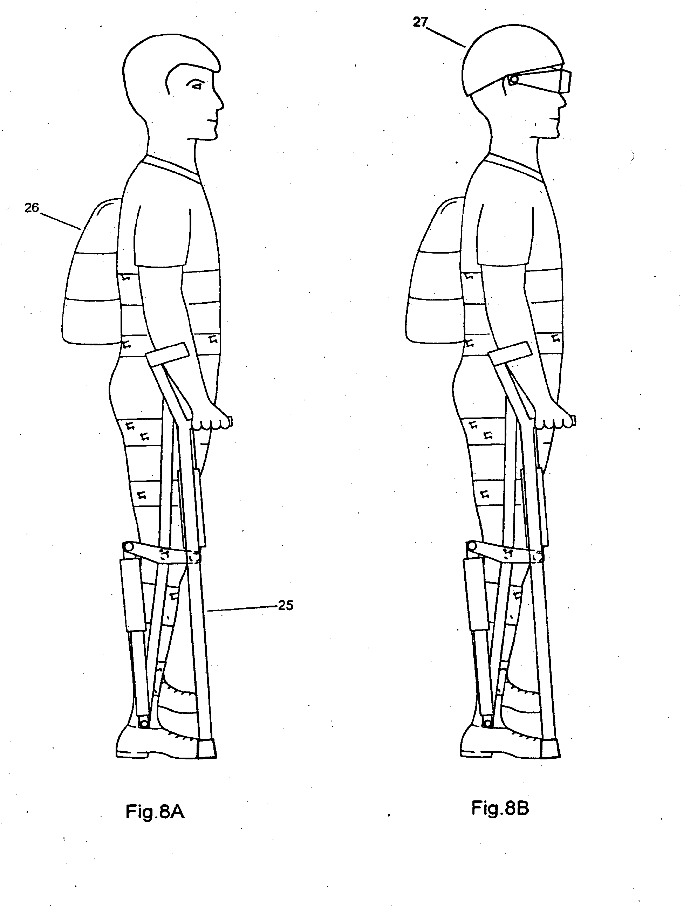

The Figures 8A and 8B show other possibilities of using the exoskeleton according to the present invention.

The Figure 8A shows in fact a patient using instead of the stroller two crutches 25 and carrying a back-pack 26 holding the mentioned electronic control unit and actuator feeding batteries, the push-buttons of the mentioned remote control being in this case provided at the grips of the crutches.

The patient in Fig. 8B is still equipped as in Fig. 8A and additionally carries in the backpack a virtual reality electronic unit that transmits the patient, through a special helmet 27, virtual reality sounds and images capable of stimulating the patient's production of adrenaline and thereby a recovery of his motor capabilities. It is in fact known that lesions or excisions of the spinal marrow due to spinal column traumas, inflammatory processes of the marrow or vertebrae, tumours or serious vascular problems may induce a paralysis of the lower limbs, owing to the fact that the lesions constitute an interruption of the nervous motor impulses deriving from the brain.

It is further known that the motor program of the limbs does not exclusively reside in the brain but also in the spinal marrow, as proved by the fact that animals can run on their legs for a few moments even after their head has been cut. The above may serve as a key for interpreting rehabilitation methods based on a program exercising the patient, in the most natural possible way, with the aid of appropriate leg supports and motor mechanisms. These methods in fact not only allow reactivating or rememorizing the legs' motor programs in the spinal marrow, but also progressively reactivating the passage of the nervous impulses originating from the brain in the damaged regions of the marrow. The usefulness of virtual reality in this case is based on the properties of adrenaline and noradrenalin to stimulate the nervous system and act as neural transporters, meaning chemical mediators that permit transmitting a nervous impulse from one neuron to another, and on the fact that the secretion of adrenaline is stimulated at the occurrence of emergency situations such as efforts, emotions, danger signals, states of arterial hypotension, asphyxia, hypothermia, etc.

Therefore it can be understood the usefulness of utilizing virtual reality during the perambulatory exercises of a tetraplegic patient, as a means for stimulating the transmission of nervous impulses in the damaged region of the marrow, and this through virtual reality images, sensations and emotions capable of stimulating an increased adrenaline level in the blood.

It is evident that numerous modifications, adaptations, variants, omissions and replacements of elements with others functionally equivalent, can be made in the examples described above without thereby going beyond the spirit of the invention and the scope of the following claims.

## Claims

1. Orthopaedic apparatus for walking and rehabilitating a motion-injured person, with an exoskeleton to support the patient's body, comprising:
- first, second and third articulated rods (1, 2, 3) set along the patient's trunk, thighs and lower legs respectively and connected by pins (4, 5) opposite the hip and knee joints,
- a pair of plates (6) set externally along one and the other knee joint, respectively,
- first and second actuators (7, 8) anchored by hinges (9, 10, 11, 12) to said plates (6) and said articulated rods (1, 3), respectively, capable of imparting motions on said rods corresponding to those of a human gait,
- an electronic control unit connected to said actuators to control their operation, so as to impart said motions to said articulated rods,
- a remote control for actuating said control unit by the patient, providing the possibility of sending, to said control unit, in addition to perambulating commands, also commands allowing the patient to assume an upright or a seated position,
- a bodice (13) fitted to the patient's trunk, of a partially rigid structure, capable of rigidly connecting the first articulated rods (1),
- means capable of clasping and rigidly fastening the first articulated rods flanking the patient's trunk to the sides of the bodice, at the time the bodice is put on by the patient,
- the hinges (10, 11) present on each of said plates (6) are flanked at the two sides of said second articulated rods (2),
**characterized in that**:
- each of said plates (6) is firmly attached to the one and the other of said second articulated rods (2) set along the patient's thighs, at a point of the rod destined to align itself with the patient's knee,
- the hinges (12) anchoring said second actuators (8) to the third articulated rods (3) are positioned at a point of the rod destined to be flanked at the side of the patient's ankle,
- said actuators are of a mechanical cylinder type, fitted internally with an electric motor, an epicycloidal reducer, and a recirculating ball screw for a linear actuation of the cylinder pushing/pulling rod or tube,
- said electronic control unit is programmed so as to command, upon receipt of a signal to move from a seated to an upright position, a progressive extension of only said first and second actuators' pushing/pulling tubes, until the articulated rods have reached an aligned position, and vice-versa to command, upon receipt of a signal to move from an upright to a seated position, the progressive re-entry of only said first and second actuators' pushing/pulling tubes, until every contiguous pair of the articulated rods has assumed a relative angular position of about 90°.

2. Orthopaedic apparatus according to the previous claim, **characterized in that** said means for removable clasping and rigidly fastening each exoskeleton rod flanked at the side of the patient's trunk to the flanks of the bodice provide that:
- the upper end of the rod (1) destined to be flanked at the side of the patient's trunk has the shape of a plate with two first loops (1 a) and a first pin (1 b), where said plate is destined to couple to a removable anchoring device present on the exoskeleton bodice;
- said removable anchoring device comprises a metallic plate (18) fastened to the bodice (13) and fitted in the front with a hollow space of adequate width to allow the entry of said upper end of the rod (1) destined to be flanked at the side of the patient's trunk, providing therein two second pins (18) capable of engaging in said first loops (1 a) of said rod (1), where the outer wall of the hollow space also provides a third loop (21) of a length adequate to allow the entry of said first pin (1b), and said anchoring device also comprises a crank (19) which can pivot around a third pin (20) and that, rotating around the latter pin, can be brought to engage said first pin by means of a recess (22), when said second pins are engaged with said first loops.

3. Orthopaedic apparatus according to claim 2, **characterized by** the presence of a belt with a buckle (16) with its ends fastened to the upper ends of the rods (3, 3a) destined to be flanked at the side of the patient's legs and capable of being tightened against the patient's knee in order to withstand the forces that the patient's lower limbs exercise on the exoskeleton at the moment of moving from the seated to the upright position and vice-versa.

4. Orthopaedic apparatus according to claim 1, **characterized in that** it provides a stroller destined to be grasped by the patient while perambulating, fitted with 4 legs two of which are resting on wheels and carrying a box (24) containing the actuators' feeding batteries and the electronic control unit for the same, while the push-buttons of said remote control are provided on the handles of the stroller.

5. Orthopaedic apparatus according to claim 1, **characterized in that** it provides as integrating means:
- a pair of crutches (25) destined to be grasped by the patients while perambulating, where the keys of said remote control are present on the handles of the crutches;
- a back pack (26) destined to be worn by the patient while perambulating, containing said electronic control unit and the actuator feeding batteries.

6. Orthopaedic apparatus according to claim 5, **characterized by** the presence, as a further integrating means, of a virtual reality electronic unit capable of transmitting the patient, through a helmet (27), virtual reality images and sounds capable of stimulating the patient's production of adrenaline while perambulating.

## Patentansprüche

1. Orthopädische Vorrichtung zum Gehen und Rehabilitieren einer in der Bewegung beeinträchtigten Person, mit einem Exoskelett zum Stützen des Körpers des Patienten, umfassend:
- erste, zweite und dritte Gelenkstangen (1, 2, 3), die entlang des Rumpfes, der Oberschenkel bzw. Unterschenkel des Patienten angeordnet und durch Stifte (4, 5) gegenüber den Hüft- und Kniegelenken verbunden sind;
- ein Paar von Platten (6), das entlang dem einen bzw. dem anderen Kniegelenk extern angeordnet ist,
- erste und zweite Stellglieder (7, 8), die durch Gelenke (9, 10, 11, 12) an den Platten (6) bzw. den Gelenkstangen (1, 3) verankert und imstande sind, den Stangen Bewegungen zu verleihen, die jenen einer Gangart eines Menschen entsprechen,
- eine elektronische Steuereinheit, die mit den Stellgliedern verbunden ist, um deren Betrieb zu steuern, so dass den Gelenkstangen die Bewegungen verliehen werden,
- eine Fernsteuerung zum Bstätigen der Steuereinheit durch den Patienten, die die Möglichkeit bietet, zur Steuereinheit zusätzlich zu Gehbefehlen auch Befehle zu senden, die dem Patienten ermöglichen, eine aufrechte oder sitzende Position einzunehmen,
- ein Mieder (13), das am Rumpf des Patienten sitzt, aus einer teilweise starren Struktur, das imstande ist, die ersten Gelenkstangen (1) starr zu verbinden,
- Mittel, die imstande sind, zu dem Zeitpunkt, zu dem das Mieder vom Patienten angelegt wird, die ersten Gelenkstangen, die den Rumpf des Patienten flankieren, an den Seiten des Mieders festzuklammern und starr zu befestigen,
- wobei die Gelenke (10, 11), die an jeder der Platten (6) vorhanden sind, die zwei Seiten der zweiten Gelenkstangen (2) flankieren,
**dadurch gekennzeichnet, dass:**
jede der Platten (6) fest an der eignen und der anderen der zweiten Gelenkstangen (2), die entlang der Oberschenkel des Patienten angeordnet sind, an einem Punkt der Stange befestigt ist, der dazu bestimmt ist, sich selbst mit dem Knie des Patienten auszurichten,
- die Gelenke (12), die die zweiten Stellglieder (8) mit den dritten Gelenkstangen (3) verankern, an einem Punkt der Stange positioniert sind, der dazu bestimmt ist, die Seite des Knöchels des Patienten zu flankieren,
- die Stellglieder von einer mechanischen Zylinderart sind, die im Inneren mit einem Elektromotor, einem Planetenuntersetzungsgetriebe und einer Kugelumlaufspindel für eine lineare Betätigung der Zylinderschub-/-zugstange oder des Zylinderschub-/-zugrohres versehen sind,
- wobei die elektronische Steuereinheit so programmiert ist, dass bei Empfang eines Signals zu einer Bewegung aus einer sitzenden in eine aufrechte Position ein Befehl zu einer fortlaufenden Verlängerung nur der ersten und zweiten Schub-/Zugrohre der Stellglieder erteilt wird, bis die Gelenkstangen eine ausgerichtete Position erreicht haben, und umgekehrt bei Empfang eines Signals zu einer Bewegung aus der aufrechten in eine sitzende Position, ein Befehl zu einem fortlaufenden Wiedereintritt nur der ersten und zweien Schub-/Zugrohre der Stellglieder erteilt wird, bis jedes angrenzende Paar der Gelenkstangen eine relative Winkelposition von etwa 90° eingenommen hat.

2. Orthopädische Vorrichtung nach dem vorangehenden Anspruch,
**dadurch gekennzeichnet, dass** die Mittel zum entfernbaren Festklammern und starren Befestigen jeder Exoskelettstange, die die Seite des Rumpfes des Patienten flankiert, an den Flanken des Mieders vorsehen, dass:
- das obere Ende der Stange (1), das dazu bestimmt ist, die Seite des Rumpfes des Patienten zu flankieren, die Form einer Platte mit zwei ersten Ausnehmungen (1a) und einem ersten Stift (1b) hat, wobei die Platte dazu bestimmt ist, mit einer entfernbaren verankerungsvorrichtung gekoppelt zu werden, die am Exoskelettmieder vorhanden ist:
- die entfernbare Verankerungsvorrichtung eine metallische Platte (18) umfasst, die am Mieder (13) befestigt und an der Vorderseite mit einem Hohlraum angemessener Breite versehen ist, so dass der Eintritt des oberen Endes der Stange (1) möglich ist, die dazu bestimmt ist, die Seite des Rumpfes des Patienten zu flankieren, wobei darin zwei zweite Stifte (18) vorgesehen sind, die imstande sind, mit den ersten Ausnehmungen (1a) der Stange (1) in Eingriff zu gelangen, wobei die Außenwand des Hohlraumes auch eine dritte Ausnehmung (21) y angemessene Länge vorsieht, so dass der EW tritt des ersten Stifts (1b) möglich ist, und die Verankerungsvorrichtung auch eine Kurbel (19) umfasst, die um einen dritten Stift (20) schwenken kann, und die, wenn sie um den letztgenannten Stift dreht, mit dem ersten Stift durch eine Vertiefung (22) in Eingriff gebracht werden kann, wenn die zweiten Stifte mit den ersten Ausnehmungen in Eingriff stehen

3. Orthopädische Vorrichtung nach Anspruch 2, **gekennzeichnet durch** das Vorhandensein eines Riemens mit einer Schnalle (16), dessen Enden an den oberen Enden der Stangen (3, 3a) befestigt sind, die dazu bestimmt sind, die Seite der Beine des Patienten zu flankieren, und der imstande ist, gegen das Knie des Patienten festgezogen zu werden, um den Kräften zu widerstehen, welche die unteren Gliedmaßen des Patienten auf das Exoskelett zum Zeitpunkt der Bewegung aus der sitzenden in die aufrechte Position und umgekehrt ausüben.

4. Orthopädische Vorrichtung nach Anspruch 1,
**dadurch gekennzeichnet, dass** sie einen Rollator umfasst, der dazu bestimmt ist, vom Patienten beim Gehen ergriffen zu werden, der mit 4 Beinen versehen ist, von welchen zwei auf Rädern ruhen, und der einen Kasten (24) trägt, der die Versorgungsbatterien für die Stellglieder und die elektronische Steuereinheit für diese enthält, während die Drucktasten der Fernsteuerung an den Griffen des Rollators vorgesehen sind.

5. Orthopädische Vorrichtung nach Anspruch 1,
**dadurch gekennzeichet**, **dass** side als integrierte Mittel vorsieht:
- ein Paar von Krücken (25), das dazu bestimmt ist, von den Patienten beim Gehen ergriffen zu werden, wobei die Tasten der Fernsteuerung an den Griffen der Krücken vorhanden sind;
- einen Rucksack (26), der dazu bestimmt ist, vom Patinten beim Gehen getragen zu werden, der die elektronische Steuereinheit und die Versorgungsbatterien des Stellgliedes enthält.

6. Orthopädische Vorrichtung nach Anspruch 5, **gekennzeichnet durch** das Vorhandensein, als weiteres integriertes mittel, einer Virtuelle-Realität-Elektronikeinheit, die imstande ist, Bilder und Töne einer virtuellen Realität, die imstande sind, die Adrenalinproduktion des Patienten beim Gehen zu stimulieren, **durch** einen Helm (27) an den Patienten zu senden.

## Revendications

1. Appareil orthopédique pour la marche et ta réhabilitation d'une personne à mobilité réduite, avec un exosquelette pour soutenir le corps du patient, comprenant :
- des première, deuxième et troisième tiges articulées (1, 2, 3) fixées le long du tronc, des cuisses et des jambes du patient, respectivement, et connectés par des broches (4, 5) au niveau de la hanche et du genou,
- une paire de plaques (6) placées à l'extérieur, le long de l'une et l'autre articulation du genou, respectivement,
- des premier et deuxième actionneurs (7, 8) ancrés par des charnières (9, 10, 11, 12) sur lesdites plaques (6) et lesdites tiges d'articulation (1, 3), respectivement, capable de conférer des mouvements sur lesdites tiges correspondant à ceux d'une démarche humaine,
- une unité de commande électronique connectée auxdits actionneurs, pour commander leur fonctionnement, de manière à générer lesdits mouvements sur lesdites tiges articulées,
- une commande à distance pour actionner ladite unité de commande par le patient, offrant la possibilité d'envoyer, à ladite unité de commande, en plus de commandes per ambulatoires, également des commandes permettant au patient d'assumer une position debout ou une position assise,
- un corsage (13) installé sur le tronc du patient, d'une structure partiellement rigide, apte à relier rigidement les premières tiges articulées (1),
- des moyens aptes à serrer et fixer de manière rigide les premières tiges articulées s'étendant le long du tronc du patient sur les côtés du corsage, au moment où le corset est placé sur le patient,
- les charnières (10, 11) présentes sur chacune desdites plaques (6) s'étendant, sur les deux côtés, le long desdites deuxièmes tiges articulées (2),
**caractérisé en ce que** :
- chacune desdites plaques (6) est reliée fermement à l'une et l'autre desdites deuxièmes tiges articulées (2) placées le long des cuisses du patient, en un point de la tige destiné à s'aligner avec le genou du patient,
- les charnières (12) ancrant lesdits deuxièmes actionneurs (8) sur tes troisièmes tiges articulées (3) sont positionnées en un point de la tige destinée à s'étendre sur le côté de la cheville du patient,
- lesdits actionneurs sont du type à cylindre mécanique, équipés intérieurement d'un moteur électrique, d'un réducteur épicycloïdal, et d'une vis à recirculation de billes pour un actionnement linéaire de la tige ou du tube de poussée / traction du cylindre,
- ladite unité de commande électronique est programmée de manière à commander, à la réception d'un signal commandant de passer de la position assise à la position debout, une extension progressive de seulement les tubes de poussée / traction desdits premier et deuxième actionneurs, jusqu'à ce que les tiges articulées soient parvenues à une position alignée, et, vice-versa, de manière à commander, à la réception d'un signal commandant de passer de la position debout à la position assise, la rentrée progressive de seulement les tubes de poussée / traction desdits premier et deuxième actionneurs, jusqu'à ce que chaque paire contigué de tiges articulées ait pris une position angulaire relative d'environ 90°,

2. Appareil orthopédique selon la revendication précédente, **caractérisé en ce que** lesdits moyens permettant l'accrochage amovible et la fixation rigide de chaque tige de l'exosquelette s'étendant le long du côté du tronc du patient jusqu'aux flancs du corsage, sont tels que :
- l'extrémité supérieure de la tige (1) destinée à s'étendre le long du côté du tronc du patient a la forme d'une plaque avec deux premières boucles (1 a) et une première broche (1b), ladite plaque étant destinée à être reliée à un dispositif d'ancrage amovible présent sur le corsage de l'exosquelette ;
- ledit dispositif d'ancrage amovible comprend une plaque métallique (18) fixée sur le corsage (13), et présentant, sur l'avant, un espace creux d'une largeur suffisante pour permettre l'entrée de ladite extrémité supérieure de la tige (1) destinée à s'étendre le long du côté du tronc du patient, incluant en lui deux deuxièmes broches (18), aptes à être engagées dans lesdites premières boucles (1a) de ladite tige (1), la paroi extérieure de l'espace creux fournissant également une troisième boucle (21) ayant une longueur suffisante pour permettre l'entrée de ladite première broche (1b), et ledit dispositif d'ancrage comprenant en outre une manivelle (19) qui peut pivoter autour d'une troisième broche (20) et qui, en tournant autour de cette dernière broche, peut être amenée à venir en engagement avec ladite première broche au moyen d'un évidement (22) lorsque lesdites deuxièmes broches sont en engagement avec lesdites premières boucles.

3. Appareil orthopédique selon la revendication 2, **caractérisé par** la présence d'une sangle avec une boucle (16) dont les extrémités sont fixées aux extrémités supérieures des tiges (3, 3a) destinées à s'étendre le long du côté des jambes du patient, et aptes à être serrées contre le genou du patient afin de résister aux forces que les membres inférieurs du patient exercent sur l'exosquelette au moment de passer de la position assise à la position debout et vice-versa.

4. Appareil orthopédique selon la revendication 1, **caractérisé en ce qu'**il inclut une poussette destinée à être saisie par le patient en per ambulatoire, munie de 4 pieds dont deux sont en appui sur des roues, et supportant un boîtier (24) contenant les batteries d'alimentation des actionneurs et l'unité de commande électronique de ceux-ci, tandis que les boutons-poussoirs de ladite commande à distance sont situés sur les poignées de la poussette.

5. Appareil orthopédique selon la revendication 1, **caractérisé en ce qu'**il inclut, en tant que moyens d'intégration :
- une paire de béquilles (25), destinées à être saisies par les patients en per ambulatoire, les touches de ladite télécommande étant présentes sur les poignées des béquilles ;
- un sac à dos (26), destiné à être porté par le patient en per ambulatoire, contenant ladite unité de commande électronique et les batteries d'alimentation des actionneurs.

6. Appareil orthopédique selon la revendication 5, **caractérisé par** la présence, en tant qu'un moyen d'intégration supplémentaire, d'une unité électronique de réalité virtuelle apte à transmettre au patient, par l'intermédiaire d'un casque (27), des images et des sons de réalité virtuelle, aptes à stimuler la production d'adrénaline du patient en per ambulatoire.
